# EUROPEAN PATENT APPLICATION

(11) **EP 2 284 747 A1**
(43) Date of publication of application: **16.02.2011**
(21) Application number: 10007779.1
(22) Date of filing: 27.07.2010
(51) Int. Cl.: G06F 19/00

(54) **Method of recording data for keeping diary of a medical testing or therapy**

(30) Priority: 12.08.2009 EP 09010374
(71) Applicant: F.Hoffmann-La Roche AG, 4070 Basel (CH); Roche Diagnostics GmbH, 68305 Mannheim (DE)
(72) Inventor: Remde, Axel, 3432 Lützelflüh (CH); Schiltges, Gilbert, 3422 Kirchberg (CH)
(74) Representative: Münch, Martin Walter

(57) **Abstract**

The invention relates to a method of recording data for keeping diary of a long-term medical testing of the glucose levels in the blood of a patient and/or of an insulin therapy of a patient suffering from diabetes. According to this method, data representing measured blood glucose levels and/or administered doses of insulin and supplementary data directly related to the measured blood glucose levels and/or administered doses of insulin are stored with a time stamp in the memory (13) of an electronic diary device (10). The supplementary data are entered into the diary device (10) by speeking voice messages into a microphone (7) of the device (10).

By the method according to the invention, typical problems of the prior art, like e.g. not correctly remembered or completely forgotten data entries, can be eliminated since data related to the measured blood glucose levels and/or administered doses of insulin are entered into the diary immediately at the time of measurement or administration in a convenient, fast and discreet way in virtually every situation. This results in a significant improvement and ease in diary keeping.

## Description

The present invention relates to a method of recording data for keeping diary of a long-term testing of the glucose levels in the blood of a patient and/or of an insulin therapy of a patient suffering from diabetes, to a diary device for keeping diary of such a long-term testing and/or therapy, to a medical device comprising the diary device and to a medical diary system comprising one or several of such medical devices according to the preambles of the independent claims.

In long-term medical testing of the glucose levels in the blood of a person and in the diabetes therapy with insulin, diary-keeping is one of the most crucial aspects. Generally, the person undergoing the testing or the therapy should keep record of all relevant events and data, like e.g. all events and data concerning blood glucose measurements, insulin administrations, meal intakes, sportive activities, illnesses, potential malfunctions of the therapy devices and the like. While there are known devices which automatically log some of those data, such as glucose measurements and insulin administrations, further valuable information is not recorded.

For diary keeping, some persons therefore use hand-written diaries. As alternative, several electronic systems are known, such as the ACCU-CHEK^{®} Pocket Compass software of Roche Diagnostics AG, Switzerland, which runs on a standard Personal Digital Assistant.

Furthermore, devices for administering of medicaments and for testing blood glucose levels are known in the art which allow for a storing of supplementary data, e.g of the patient's lifestyle, as well as of therapy related data in a memory of the device.

EP 2 060 284 A1 discloses a device for the self-administration of liquid drugs in adjustable doses which beside a dosing module for administering the liquid drug comprises an electronics module with a memory into which data related to the dosed amount of the liquid drug and therapy related data can be stored together with a time stamp. The dosed amount of liquid drug is automatically stored when the user administers the drug while the other therapy related data are entered manually into the device.

EP 1 369 688 A2 discloses a testing device for testing the glucose levels in the blood of persons. While the measured glucose values are automatically stored in the device, supplementary data, like e.g. food consumption, medication intake and physical exercise, must be entered manually.

In practice, diaries and reports generated with either of the above methods and devices often are incomplete and/or show errors. Many persons do not keep a diary at all because they feel it to be too cumbersome in everyday life. It is estimated by medical doctors that typically 40% of all diary entries are missing, incomplete or wrong.

Therapy diaries, however, are of major importance for health care professionals for monitoring the therapy and for long-term optimization. In some cases, they are also required by law for proving the therapy quality (in many countries, for example, for car drivers).

Thus, it is the objective of the present invention to improve the quality of medical testing and therapy diaries in the field of treatment of diabetes, and thereby the quality of the therapy as a whole while putting minimum effort on the person undergoing the testing or therapy.

This objective is achieved by the method of recording data for keeping diary, by the diary device, by the medical device and by the medical diary system according to the independent claims.

One aspect of the invention relates to a method of recording data for keeping diary of a long-term testing of the glucose levels in the blood of a patient and/or of an insulin therapy of a patient suffering from diabetes. According to this method, data representing the measured blood glucose levels and/or the administrated doses of insulin and supplementary data directly related to the measured blood clucose levels and/or administrated doses of insulin, like e.g. information about the reason for an bolus administration of insulin or a comment to a measured blood glucose level, are stored with a time stamp, in the memory of an electronic diary device. The supplementary data are stored together with an own time stamp or together with the time stamp of the measured blood glucose level and/or administrated dose of insulin they relate to. In case the supplementary data are stored together with an own time stamp, it is preferred that they are linked to the data the relate to by pointers, corresponding identifiers, or the like such that they can be easily combined for later presentation and evaluation.

The time stamp preferably includes the date and the time of entry of the data into the diary device or of storing of the data in the memory of the device.

At least some of the supplementary data and preferably all supplementary data which are to be stored in the memory are entered into the diary device by speeking voice messages into a microphone of the device.

By the method according to the invention, typical problems of the prior art, like e.g. not correctly remembered or completely forgotten data entries, can be eliminated since the supplementary data are entered into the diary immediately at their occurrence in a convenient, fast and discreet way in virtually every situation. This results in a significant improvement and ease in diary keeping.

In a preferred embodiment of the method, in addition supplementary data not directly related to the measured blood glucose levels and/or an administrated doses of insulin, like e.g. data relating to food intake in general, physical activities, illnesses or ingestion of further medicaments, are stored, together with time stamps, in a memory of the electronic diary device. The memory in which these data are stored can be the same memory in which the data directly related to the measured blood glucose levels and/or an administrated doses of insulin are stored in or can be a separate memory.

The time stamp again preferably includes the date and the time of entry of the data into the diary device or of storing of the data in the memory of the device.

At least some of the supplementary data not directly related to the measured blood glucose levels and/or an administrated doses of insulin and preferably all of these supplementary data which are to be stored in the memory are entered into the diary device by speeking voice messages into a microphone of the device.

In a further preferred embodiment of the method, the voice messages entered into the diary device are stored together with a time stamp in the memory so that they can be played back for checking and/or analyses.

In still a further preferred embodiment of the method, the voice messages are, preferably automatically, transformed into text by the diary device and are then stored as text together with a time stamp in the memory. This can be done instead of storing the voice messages in the memory or in addition to storing the voice messages. Tranforming the voice messages into text significantly facilitates automated analyses of the stored data.

In yet a further preferred embodiment of the method, the voice messages entered into the diary device comprise key words and/or numeric values which by means of the diary device are identified, transformed into text and stored as text together with a time stamp in the memory of the diary device. Instead of storing key words as text they may also be stored in a code. For example, key words may be stored in the device in form of a list and the device may store the order number of the key word in the list rather than the text of the key word as such. Storing only the identified keywords and/or numeric values of a voice message as text or code together with a time stamp provides the advantage that automated analyses of the stored data are further facilitated and that the memory requirements are considerably reduced as compared to storing the voice messages as such.

In yet a further preferred embodiment of the method, the text generated by the diary device through transformation of the voice messages or of the keywords and/or numeric values identified in the voice messages in each case is checked and confirmed by the user before it is stored in the memory of the device. By this, the diary can be kept free of data errors in a simple and convenient way.

In yet a further preferred embodiment of the method, the diary device is at least partially controlled by the voice of the user. By this, diary keeping becomes even more discrete and convenient.

If the diary device used for storing the data comprises or is connected with means for testing the glucose level in the blood of a patient, it is preferred that, upon a testing of the glucose level, the test result is automatically stored in the memory of the diary device together with a time stamp. Preferably, in addition to the test result, a voice message related to the test result and/or a transcription into text of such a voice message is stored in the memory, together with a separate time stamp or with the related test result.

Alternatively or additionally, if the diary device used for storing the data comprises or is connected with means for administration of insulin, preferably with an insulin pump or an insulin pen, it is preferred that, upon an administration of insulin, the dose of insulin administered is automatically stored in the memory of the diary device together with a time stamp. In case of a therapy with an insulin pump it might be preferred to store only the unscheduled administration ("bolus" administration), since the scheduled administration profile ("basal" administration profile) is known and thus normally does not need to be monitored for diary-keeping purposes. Also here it is preferred that in addition to the dose, an explanatory voice message related to the dose and/or a transcription of such a voice message into text is stored in the memory, together with a separate time stamp or with the related dose.

Through this automated storing of data related to the testing or to the therapy, diary keeping is significantly facilitated.

In the two before mentioned embodiments of the method it is furthermore preferred that the means for testing the glucose level and/or the means for administration of insulin are controlled via the diary device.

Furthermore, alternatively or additionally it is preferred that, based on supplementary data, on the time of day and/or on a result of testing the glucose level, a specific amount of insulin to be administered is calculated and the administration thereof is initiated via the diary device, wherein it is preferred that at least some of the data used for the calculation have been entered by voice message into the diary device. Preferably, in addition a voice message related to the insulin administration and/or a transcription of such a voice message into text is stored in the memory, together with a separate time stamp or with the related insulin administration data.

By this, the testing and/or therapy as such are significantly facilitated for the patient.

In yet a further preferred embodiment of the method, informations about malfunctions or defects of the diary device and/or of connected devices and means, like connected means for testing the glucose level in the blood of a patient and/or means for administration of insulin, are stored in the memory of the diary device together with time stamps. Preferably, further explanatory informations are entered as voice messages into the diary device and are stored in its memory as voice message and/or text, together with a separate time stamp or with the related malfunction or defect.

This helps avoiding misinterpretations when analysing the data stored in the memory of the diary device.

In yet a further preferred embodiment of the method, the data stored in the diary device are downloaded to an external device, preferably to a personal computer, for analysing said data. At least some of these data have been entered into the device by speeking voice messages.

By this it becomes possible to keep the diary device, which preferably is a portable device, small and simple and to perform extensive analyses of the data and visualisation of the results on a computer which can be stationary.

A second aspect of the invention relates to a diary device for keeping diary of a long-term testing of the glucose levels in the blood of a patient and/or of an insulin therapy of a patient suffering from diabetes. Preferably, the diary device is suitable for use in the method according to the first aspect of the invention. The diary device comprises a memory for storing data representing measured blood glucose levels and/or representing administrated doses of insulin, in particular bolus insulin administrations, and for storing supplementary data directly related to the measured blood glucose levels and/or administrated doses of insulin, like e.g. information about the reason for an bolus administration of insulin or a comment to a measured blood glucose level, together with a time stamp. The supplementary data can be stored together with their own time stamps or together with the time stamps of the measured blood glucose levels and/or administrated doses of insulin they relate to.The time stamp preferably includes the date and the time of entry of the data into the diary device or of storing of the data in the memory.

The diary device furthermore comprises a clock circuitry, for generating the time stamps, and a voice recording unit, for entering at least a part of the supplementary data directly related to the measured blood glucose levels and/or administrated doses of insulin, which are to be stored in the memory of the diary device, by speeking voice messages.

With the diary device according to the invention, typical problems of the prior art, like e.g. not correctly remembered or completely forgotten data entries, can be eliminated since supplementary data related to a measured blood glucose level and/or an administrated dose of insulin are entered into the diary immediately at the time of measurement or administration, respectively, in a convenient, fast and discreet way in virtually every situation. This results in a significant improvement and ease in diary keeping.

In a preferred embodiment of the diary device, it further comprises a memory for storing in addition supplementary data not directly related to the measured blood glucose levels and/or an administrated doses of insulin, like e.g. data related to food consumption, physical activities, illnesses and intake of further medicaments of the patient, together with time stamps. In that case it preferred that is furthermore comprises a voice recording unit for entering at least a part of the supplementary data not directly related to the measured blood glucose levels and/or an administrated doses of insulin, which are to be stored in the memory (13, 16, 18), into the diary device (10) by speeking of voice messages.

Preferably, the diary device is designed in such a manner that it allows one-hand operation when entering voice messages.

In a further preferred embodiment of the diary device, the device is adapted to allow storage of voice messages entered via the voice recording unit together with time stamps, which allows playback for later checking and conversion into text of the voice messages.

In yet a further preferred embodiment, the diary device comprises a playback unit with a loudspeaker or the like. The playback unit maybe used for playing back the voice messages, but also for indicating the order in which information should be entered. The loudspeaker or the like may also be used as general indication device for reminder alerts, indicating device errors and the like.

In still a further preferred embodiment of the diary device, it further comprises a text recognition unit for voice-to-text transformation of the entered voice messages into text to be stored in the memory with time stamps. Preferably, the transformation takes place automatically when a voice message has been entered. Transforming the voice message into text facilitates analyses of the stored data.

In yet a further preferred embodiment of the diary device, the text recognition unit is adapted to only identify and transform certain keywords and/or numeric values contained in the entered voice messages into text to be stored in the memory together with time stamps.

In the above case it is furthermore preferred that this text recognition unit is adapted to learn new keywords by playing back voice messages stored in the memory and entering the corresponding text via an input unit of the diary device into the memory. By this it becomes easy to configure the diary device to the requirements and language of the user.

Preferably, in the two before mentioned embodiments, the diary device in configured in such a manner that the text resulting from the transformation must be confirmed by the user before it is stored, together with a time stamp, in the memory.

For this, the diary device preferably comprises a display and one or more control buttons and is designed in such a manner that the text resulting from the transformation performed by the text recognition unit is displayed for rechecking by the user and can be stored in the memory together with a time stamp by pushing one or several of the control buttons for confirmation.

By this, the diary can be kept free of errors resulting from a wrong transformation in a simple and convenient manner.

In yet a further preferred embodiment, the diary device is designed in such a manner that at least partially it can be controlled by the voice of the user.

By this, diary keeping becomes even more discrete and convenient.

In yet a further preferred embodiment, the diary device further comprises a data interface for downloading the data stored in the memory to an external device, preferably to a personal computer, for analysing said data. By this it becomes possible to keep the diary device, which preferably is a portable device, small and simple and to perform extensive analyses of the data and visualisation of the results on a computer which can be stationary.

A third aspect of the invention relates to a medical device, which comprises a diary device according to the second aspect of the invention and further comprises means for testing the glucose level in the blood of a patient and/or means for administration of a insulin, preferably an injection module for insulin, like an insulin pen or a insulin pump. The diary device is linked together with the means for testing the glucose level and/- or with the means for administration of insulin in such a manner that, upon a testing of the glucose level and/or upon an administration of insulin, the test result and/or the dose of insulin can be stored in the memory of the diary device in each case together with a time stamp, and preferably additionally together with a voice message related to the test result or the insulin dose and/or with a transcription into text of such a voice message.

By the medical device according to the invention, diary keeping in case of long-term testing and/or therapy is further facilitated and improved.

In cases of a diabetes therapy with an insulin pump, it might be preferred to store only the unscheduled administrations ("bolus" administrations), since the scheduled administration profile ("basal" administration profile) is known and thus does not need to be documented for diary-keeping purposes.

The storing preferably takes place automatically, so that the storing of data related to the testing or to the therapy for the diary keeping is significantly facilitated and can not be forgotten.

In a preferred embodiment of the medical device, the diary device and the means for testing the glucose level in the blood of a patient and/or the means for administration of insulin are contained in separate housings and are linked with each others by interfaces, which preferably are wireless.

In a further preferred embodiment of the medical device, in which it comprises at least means for administration of insulin, which preferably are embodied as an injection module for insulin, and preferably also comprises means for testing the glucose level in the blood of a patient, the medical device comprises means for the computation of a dose of insulin to be administered based upon supplementary data and/or based on the result of testing the glucose level stored in the memory of the diary device. Preferably, the computation of the insulin dose is at least partially based on stored data which have been entered as voice messages.

In yet a further preferred embodiment, in which it comprises means for administration of insulin and/or means for testing the glucose level in the blood of a patient, the medical device is designed in such a manner that the means for testing the glucose level and/or the means for administration of insulin can be controlled from a housing containing the diary device.

By this, the testing and/or therapy as such is significantly facilitated.

In yet a further preferred embodiment, the diary device of the medical device is adapted to store informations about malfunctions and/or defects of the diary device, of the means for testing the glucose level in the blood of a patient and/or of the means for administration of insulin in its memory together with time stamps. Preferably, it is furthermore adapted for entering additional explanatory informations to the informations about malfunctions or defects as voice messages and for storing these voice messages as such and/or as text, together with separate time stamps or with the related information about malfunction or defect, in the memory (13, 16, 18) of the diary device (10).

This helps avoiding misinterpretations when analysing the data stored in the memory of the diary device.

A fourths aspect of the invention relates to a medical diary system comprising one or several diary devices and/or medical devices according to the second and the third aspect of the invention and further comprising an external unit to which the data stored in the memories of the diary devices can be downloaded by means of a wired or wireless interface, for analysing said data. By this it becomes possible to keep the diary devices or medical devices, respectively, which preferably are portable devices, small and simple and to perform extensive analyses of the data and visualisation of the results on a computer which can be stationary.

Further preferred embodiments of the invention become apparent from the dependent claims and from the following description by way of the drawings. Therein show:
Fig. 1 a front view of a first embodiment of the medical device according to the invention;
Fig. 2 a simplified structural diagram of the medical device of Fig. 1;
Fig. 3 a front view of a second embodiment of a medical device according to the invention;
Fig. 4 a simplified structural diagram of the medical device of Fig. 3;
Fig. 5 shows an example of a display indication of the device according to Figures 3 and 4 showing a table-like data diary; and
Fig. 6 a simplified structural diagram of a medical diary system according to the invention.

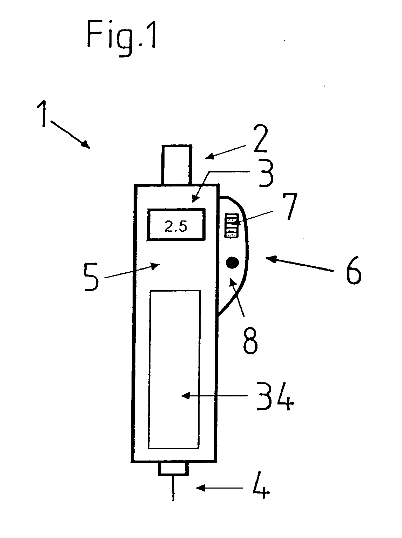

Fig. 1 shows a first medical device 1 in accordance with the present invention which is embodied as an insulin pen.

For injecting a dose of insulin, an unlocking button (not visible) on the housing 5 is pressed, thus moving the dose setting knob 2 from a retracted position inside the housing 5 to the shown projecting position. By rotating the dose setting knob 2, the dose amount of insulin is set and indicated on a dose scale 3, e.g. a mechanical or electronically digital scale. By manually pressing the dose setting knob 2 down and bringing it back into its retracted position, the insulin dose is administered from an insulin cartridge inside the housing 5 via the detachable single-use cannula 4. The filling level of the cartridge is visible through a cartridge window 34 arranged at the front of the housing 5.

The medical device 1 additionally comprises a voice recording unit 6 for recording voice notes via a microphone 7.

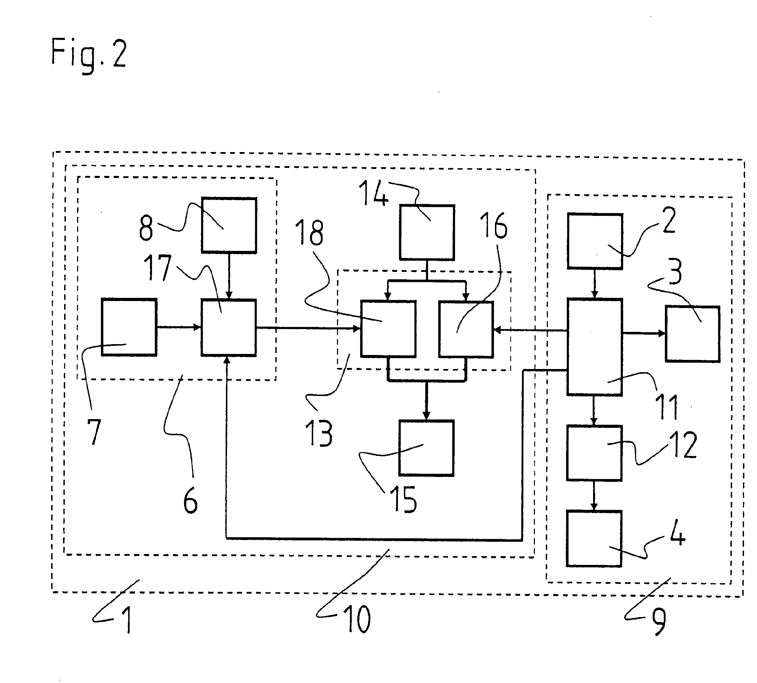

Further components of the medical device 1 as well as the interaction of the single components are best understood from Fig: 1 in combination with Fig. 2. Fig. 2 shows a simplified structural view of the medical device 1.

For clarity reasons, the medical device 1 is separated into an injection module 9 and an electronics module 10. The electronics module 10 as such forms a diary device according to the claims and in combination with the injection module 9 forms a medical device according to the claims. The injection module 9 comprises a dosing knob 2, a dosing mechanism 11, a drug cartridge 12, a cannula 4, and a dose scale 3. The dose scale 3 may be a mechanical digital display as shown in Fig. 1 or an analogue indicator which operates, for example, according to the principal of a micrometer screw. It may further be an electronic dose display and be comprised by the electronics module 10, which is further described below. For the overall design of the injection unit 1, a large variety of reusable and disposable designs is known in the art.

The electronics module 10 comprises a memory 13, a clock circuitry 14, a data interface 15, and a voice recording unit 6. When a drug dose is administered, the corresponding dose amount is stored in a dose memory 16 along with a time stamp generated by the clock circuitry 14. For this purpose, the dosing mechanism 11 comprises an electronic encoder as known in the art for transforming the dose which is entered by rotating the dosing knob 2 into an electrical signal. Storing the dose amount in the dose memory 16 is performed when, after the administration of the dosed amount, the dose setting knob 2 again enters its fully retracted locking position, thereby indicating that a drug administration is completed.

In addition to storing the dose amount in the dose memory 16, the voice recording unit 6 is automatically activated. The voice recording unit 6 comprises a microphone 7 and a processing unit 17. The electrical signal, which is generated by the microphone 7 upon the user speaking a voice message into the microphone 7, is amplified, digitized and further processed by the processing unit 17. The processing unit 17 comprises components and implements functionality as known from state-of-the-art digital dictating devices. The digitized voice message is stored in a voice memory 18 along with a separate time stamp which is generated by the clock circuitry 14 or together with the time stamp of the admininstered drug dose it relates to. The voice recording unit 6 advantageously implements a voice controlled activation (VCA) as known from dictating devices, such that recording is only activated if and as long as the user actually enters a spoken message. The voice memory 18 advantageously stores voice messages in a memory-efficient format which is suited for storing voice recordings, such as the "dds" format or another suited format known in the art.

Automatically activating the voice recording unit 6 upon completion of a drug injection allows the person with diabetes to add voice messages with supplementary information for every administration without requiring additional handling steps. Besides drug administration, the person with diabetes may start and stop the recording at any time via the manual recording button 8 in order to enter further voice messages. Those voice messages are stored in the voice memory 18 along with a time stamp, too.

If the medical device 1 is, besides the replaceable cartridge 12 and the single-use cannula 4, designed as durable device, the electronic module 10 and in particular the voice recording unit 6 may be comprised by the housing 5 of the medical device 1. Alternatively, the whole injection module 9 may be designed for single use and the electronic module 10 may be designed as durable unit which is releasably attached to the injection module 9.

For diary-keeping purposes, the data stored in both the dose memory 16 and the voice memory 18 may be downloaded to an external device via the data interface. The date interface is advantageously a wireless RF interface, such as a Bluetooth interface 15, but may also be an IR interface or a wired interface, such as a USB interface. The external device to which the data are transferred may be a PC which runs a corresponding diary software or a dedicated diabetes management device similar to a personal digital assistant (PDA). The data interface is additionally used to upload parameters, such as the date and time of day, to the medical device 1.

In an alternative embodiment, the medical device 1 according to the Figures 1 and 2 may also comprise a playback unit for playing back of voice messages stored in the voice memory 18.

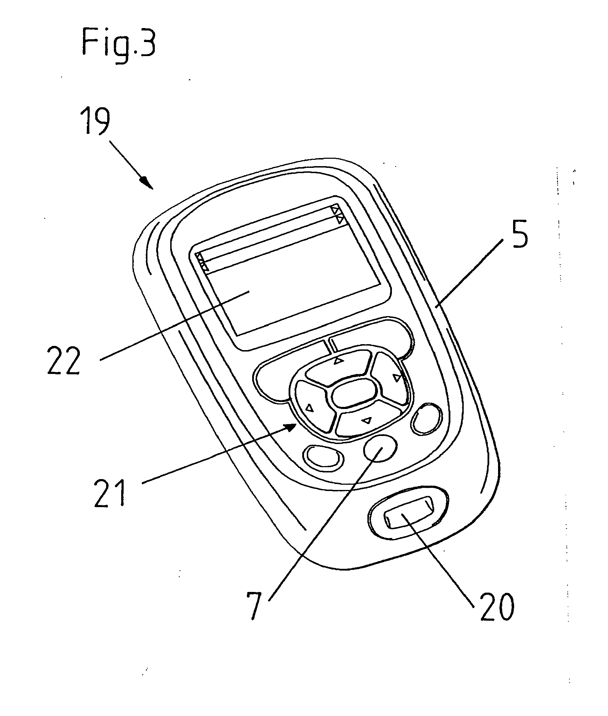

Fig. 3 shows a medical device according to the invention which is embodied as a diabetes management device 19. The diabetes management device 19 integrates the functions of a blood glucose meter, a remote controller for an insulin pump as well as an electronic diabetes diary with data presentation and analysis functionality. The diabetes management device 19 comprises a test strip socket 20 into which the person with diabetes may insert a blood glucose test strip in accordance with a variety of state-of-the-art devices, for example as available from Roche Diagnostics AG, Switzerland, under the name ACCU-CHEK^{®} Aviva. Alternatively to a test strip socket 20, the device 19 may itself carry a number of test strips, for example in a disposable drum or on a flexible tape. The test strips being used one after the other. Corresponding devices are available Roche Diagnostics AG, Switzerland, under the names ACCU-CHEK^{®} Compact plus or ACCU-CHEK^{®} Mobile.

The glucose meter is operated via a keyboard 21 and the results are shown on a display 22. An insulin pump which is carried by a diabetic person for the insulin therapy can be remotely controlled via the keyboard 21 and the display 22 as well. For this purpose, the diabetes management device 19 comprises a pump interface 26 (not visible in Fig. 3) which is typically made by a RF interface or another suited wireless technology. Blood glucose measurements, insulin administration as well as further therapy-relevant information, such as insulin pump errors, are stored in the diabetes management device 19 for diary-keeping purposes. The display 22 may especially be a graphical display and allows presentations of the data in an appropriate form for analysis and interpretation, such as curves of blood glucose values over time, time scales indicating insulin administrations, several types of statistical analysis, and the like. The diabetes management device 19 further comprises a microphone 7 for entering voice-messages for diary-keepingpurposes and advantageously also for controlling dedicated functions of the diabetes management device 19 and/or an insulin pump by voice as will be described in more detail in the following.

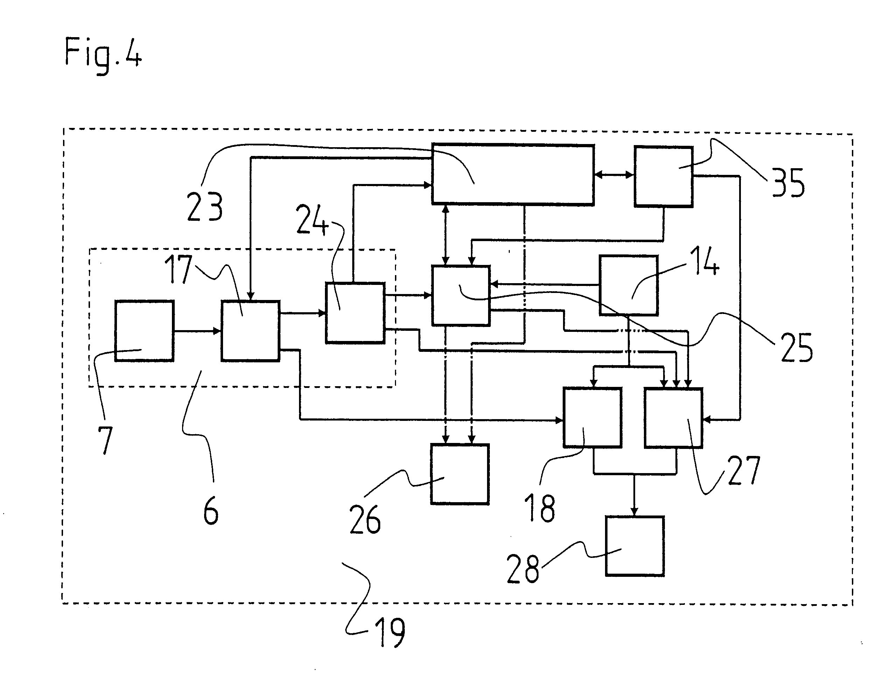

Fig. 4 shows a simplified structural view of the diabetes management device 19 as shown in Fig. 3. It has a user interface 23 comprising the keyboard 21, the display 22, as well as non-visual indicators, such as a buzzer and/or a pager vibrator.

The voice recording unit 6 of the diabetes management device 19 comprises a microphone 7 and a processing unit 17 as described above. Voice messages are stored in a voice memory 18 as described above with reference to Fig. 1 and Fig. 2. The voice recording unit 6 may be activated at any time via the user interface 23 for entering a voice message. Additionally, the voice recording unit 6 is automatically activated if the person with diabetes performs a blood glucose measurement with a glucose measurement modul 35 which is incorporated within the diabetes management device 19 in order to allow easy entry of further specific information with respect to the measurement and/or a measured value.

The voice recording unit 6 further comprises a text recognition unit 24 which is coupled to the processing unit 17 for automated voice-to-text transformation of a selected number of keywords and/or of numeric values. Keywords may be used for simple entries of typical and frequent comments, such as "sports", "hypoglycemia", "stress", "dinner", "snack", and the like.

The text recognition unit 24 is operatively coupled to a bolus computation unit 25. The bolus computation unit 25 allows to automatically compute an appropriate insulin bolus which should be infused by the insulin pump based on the time of day, the size of a meal and/or a recently measured blood glucose value. Bolus computation may further consider the therapy history over the last few hours based on therapy data stored in the data memory. Exemplary bolus computation units are disclosed, among others, in WO 2006/066926 A1.

A bolus which is computed by the bolus computation unit 25 is displayed by the display 22, such that the person with diabetes can confirm or modify it via the keyboard 21. After the acknowledgement, the bolus delivery command is transmitted to an insulin pump via the pump interface 26.

Coupling the bolus computation unit 25 with the text recognition unit 24 allows a person with diabetes to enter a meal size in a very convenient and discrete way by simply speaking a corresponding numeric value, such as an estimated number of grams of carbohydrate or carbohydrate exchange units which is used as input value for the bolus computation. Alternatively or additionally to carbohydrate information, further characterizing information, such as the protein and fat content and the relative amounts of liquid to solid components may be entered, stored, and processed in an analogue way. In embodiments where the diabetic may enter several information that shall be stored and/or processed separately, the device 19 advantageously indicates the order in which the data should be entered, for example via corresponding text messages on a device display 22. The patient may enter the data accordingly via voice messages and separate the different data, for example, by pressing a button each time a new data has been entered. Additionally, the patient may enter the data in an arbitrary order and start each entry with a corresponding keyword which may be recognized by the text recognition unit 24.

The numeric value as recognized by the text recognition unit 24 and the insulin bolus amount which is to be infused are stored in the data memory 27. The voice message itself is stored in the voice memory 18 in the same way as further voice notes. Alternatively to entering meal information via the voice recording unit 6, the person with diabetes may directly enter the information via the user interface 23 and/or may directly remotely control the insulin pump via the user interface 23 if he prefers so in a specific situation.

Even if meal size information is entered via the voice recording unit 6, the person with diabetes may be required to initiate the entry via the user interface 23 in advance for safety purposes.

Comments which are entered via the voice recording unit 6 and are automatically transformed to text by the text recognition unit 24 are advantageously displayed and/or may be modified via the user interface 23 prior to storing them in the data memory 27.

The information which is stored in both the voice memory 18 and the data memory 27 may be transmitted to an external computer, such as a PC, via a computer interface 28, such as an IR or a USB interface. The computer interface 28 may alternatively be integral with the pump interface 26. The computer interface 28 may be further used for configuration of the diabetes management device 19, setting the time and date of the clock circuitry 14, and the like.

The diabetes management device 19 may additionally comprise a food database (not shown) which is operatively coupled to the bolus computation unit 25. Such a food database stores the absolute or relative carbohydrate amounts of diverse foods (for example for fruits, bread, pasta and the like as carbohydrate amount per 100 grams, for pizza, hamburgers, and the like as "small", "medium", "large"). The text recognition unit 24 is advantageously configured to recognize the meal type and meal size information which is stored in the food database. In this way, the person with diabetes does not need to do any manual calculations or to look up information in lists and tables. The combination of voice recording unit 24, bolus computation unit 25 and food database allows him or her to enter the required information in a very simple way, e.g. by just speaking "pizza small", "potatoes 150 grams", or the like.

In somewhat further advanced embodiments, the voice recording unit 6 with integrated text recognition unit 24 can be used for generally controlling the operation of the diabetes management device 19 and/or an insulin pump beside using it for entering voice notes and meal size information. In a somewhat simplified embodiment, the voice recording unit 6 does not comprise a text recognition unit 24 and is only used for the convenient entry of voice messages in a similar way to the medical device 1 as shown in Fig. 1 and Fig. 2.

In an alternative embodiment, the medical device 19 according to the Figures 3 and 4 may also comprise a playback unit for playing back of voice messages stored in the voice memory 18.

For clarity reasons, Fig. 4 shows only those components which are of relevance in a framework of the present invention. Further components, such as a power supply and a controller unit which controls the overall operation at the device 19, are not shown but obvious for a person skilled in the art. Several functional components of the device 19 may be realized in an integrated way using known and/or applications-specific electronics circuitry.

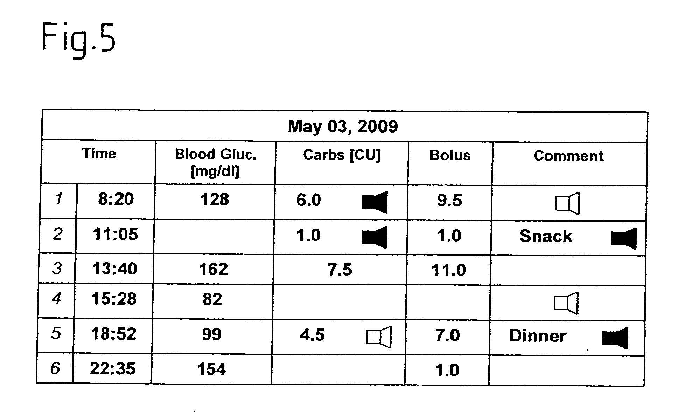

Fig. 5 illustrates how the data which are entered and stored by the diabetes management device 19 of Fig. 3 and Fig. 4 may be presented in a table-like diary. It has to be understood that Fig. 5 is exemplary. Dependent on the processing and displaying capabilities of the device 19, a large variety of tables, graphs, and mixed presentations may be used. Further data which are not shown in table 5, such as temporary adjustments of an insulin pump, may be displayed as well.

According to the entry in Line 1, the diabetic measured a blood glucose value of 128 mg/dl at 8:20 and administered an insulin bolus of 9.5 IU (International Units) for compensating the intake of 6.0 CU (Carbohydrate Units) of carbohydrates. International Units and Carbohydrate Units are well known in the field of diabetes therapy as measures for insulin amounts and carbohydrate amounts, respectively. The bolus amount is advantageously computed by the bolus computation unit 25, based on the entered carbohydrate intake and potentially also the blood glucose value.

The filled loudspeaker symbol in the "Carbs" cell indicates that the carbohydrate amount was entered via the voice recording unit 6 and transformed into numeric information by the text recognition unit 24. By selecting a cell which shows a loudspeaker symbol via the keyboard, the recorded voice information can be played back for verification purposes. Generally, a filled loudspeaker symbol indicates a voice message which was successfully processed by the text recognition unit 24.

The loudspeaker symbol in the "Comment" cell of Line 1 indicates that the diabetic entered a further voice message, for example a further meal characterization. The loudspeaker symbol is unfilled because the voice message could not be transformed into text by the text recognition unit 24. By selecting the cell, however, the voice message may be played back.

Line 2 indicates that the diabetic ate a snack meal at 11.05. Here, the loudspeaker symbol in the "Comment" cell is filled, indicating that the word "Snack" was entered as voice message and automatically was transformed into text by the text recognition unit 24.

Line 3 comprises no cells with loudspeaker symbol, thus indicating that the diabetic manually entered the carbohydrate information via the keyboard 21 and did not enter a further voice comment.

In Line 5, the loudspeaker symbol in the "Carbs" cell is not filled. An unfilled loudspeaker symbol in the "Carbs" cell indicates that a corresponding voice message is present which, however, does not correspond to the numeric carbohydrate information. This may be the case if the text recognition unit 24 could not successfully transform the voice message into text, e.g. due to acoustic distortions, or the diabetic decided to manually modify the value after entry. The entry "Dinner" in the "Comment" cell was entered via the voice recording unit 6 and successfully transformed into text by the text recognition unit 24.

If the diabetes mamagement device 19 is configured to evaluate and/or analyze the data, it may further be configured to actively request the diabetic to retrospectively add further voice comments if the data indicate an unusual and/or exceptional situation that may need further explanation. Typical examples are hypoglycemic or hyperglycemic events, the delivery of a bolus of unusual size and/or time, an unexpectedly raised glucose value after a meal, or the like.

If the diabetes management device 19 is configured to be coupled to further devices, such as an insulin pump, the device 19 may further request the diabetic to enter comments on alarms, warnings or errors which are or have been generated by this device. A typical example would be an occluded infusion cannula or a device error.

While a handheld diabetes management device 19 as shown in Fig. 3 is designed to be carried by the diabetic in virtually every situation and incorporates fundamental diary and evaluation functions, its processing, storing and displaying capabilities are limited. In addition, many diabetics use more than one device in parallel, each device generating and storing therapy-related data which are valuable for diary-keeping and therapy evaluation purposes.

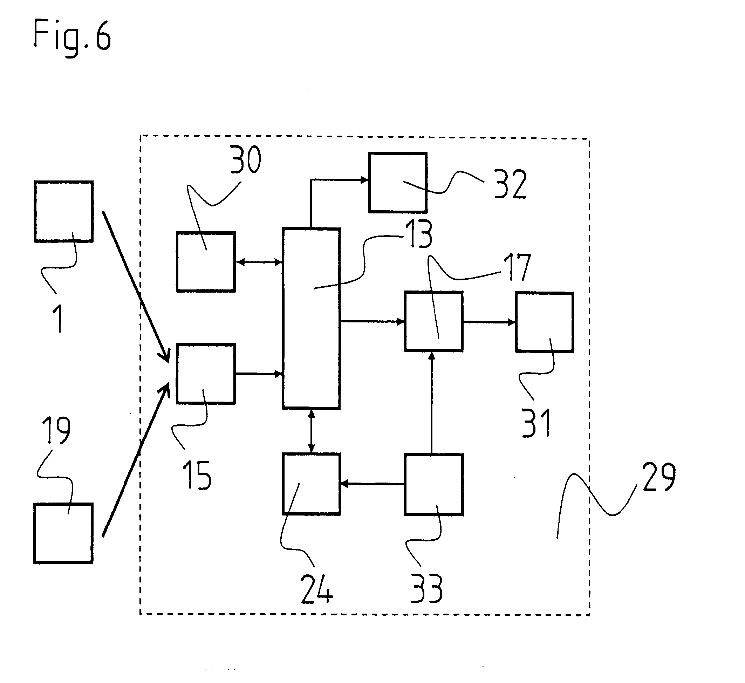

Therefore, diary devices and/or medical devices according to the present invention may favorably be used in combination with an external device 29 to form a system according to the invention as shown in Fig. 6. The external device 29 may either be a dedicated device or may be implemented by means of a standard computer, such as a PC and/or as described below.

The external device 29 of Fig. 6 comprises one or multiple data interface 15 for coupling to diary devices and/or medical devices 1, 19 such as insulin pens, insulin pumps, blood glucose meters and diabetes management devices. At least one of the diary devices and/or medical devices is designed in accordance with the present invention and is, for example, a medical device 1 according to the Figures 1, 2 or according to the Figures 3, 4. The data interface 15 may be realized by known means and may, for example, be an USB interface, a wired or wireless RS 232 interface, a Bluetooth RF interface, or the like. The data transmitted from the diary devices and/or medical devices 1, 19 are stored in a memory 13, in particular a volatile semiconductor memory. For permanent storage, an additional storage unit 30, such as a hard disk, is advantageously provided. A processing unit 17 is operatively coupled to the memory 13 and to an output unit 31, such as a graphic display or computer monitor. The processing unit 17 processes and evaluates the data stored in the memory 13 to generate diary tables, diagrams, charts, statistical evaluations and the like, for presentation via the output unit 31.

The external device 29 further comprises a text recognition unit 24 and a playback unit 32. These units, in combination, allow automated transformation of voice messages into text even if the diary device or medical devices 1 does not include a text recognition unit 24, such as the exemplary medical device 1 of Figures 1, 2. Even if a diary device or a medical device comprises a text recognition unit 24, its capabilities are typically limited. In contrast, the text recognition unit 24 of the external device 29 can be more powerful. It can be used, for example, to transform voice messages which could not be transformed by a text recognition unit 24 of a diary device and/or medical device 19 as explained in the context of Fig. 5.

In addition, the text recognition unit 24 of the external device 29 may be configured to "learn" new vocabulary by playing a voice message back and subsequently entering the corresponding text via an input unit 33 of the external device 29. The input unit 33 may, for example, comprise a keyboard, a mouse, a touch screen, or the like. The input unit is further used for the overall controlling of the diary devices and/or medical devices 1, 19.

After teaching vocabulary into the text recognition unit 24, it may optionally also be transferred to the text recognition unit 24 of a diary device or medical device 19 via the data interface 15.

The text recognition unit 24 of the external device 29 may be a dedicated hardware and/or software module or may make use of commercially available text recognition software, such as "Dragon Naturally Speaking" as provided by Nuance Communications, Inc, Burlington , MA, USA.

While there are shown and described presently preferred embodiments of the invention, it is to be distinctly understood that the invention is not limited thereto but may be otherwise variously embodied and practiced within the scope of the following claims.

## Claims

1. Method of recording data for keeping diary of a long-term testing of the glucose levels in the blood of a patient and/or of an insulin therapy of a patient suffering from diabetes, wherein data representing measured blood glucose levels and/or administrated doses of insulin and supplementary data directly related to the measured blood glucose levels and/or administrated doses of insulin are stored, together with separate or common time stamps, in a memory (13, 16, 18) of an electronic diary device (10) and wherein at least some of the supplementary data are entered into the diary device (10) by speeking voice messages in a microphone (7) of the diary device.

2. Method according to claim 1, wherein in addition supplementary data not directly related to the measured blood glucose levels and/or an administrated doses of insulin are stored, together with time stamps, in a memory (13, 16, 18) of the electronic diary device (10) and in particular, wherein at least some of these supplementary data not directly related to the measured blood glucose levels and/or an administrated doses of insulin are entered into the diary device (10) by speeking voice messages in a microphone (7) of the diary device.

3. Method according to one of the preceding claims, wherein the voice messages are stored, together with time stamps, in the memory (13, 16, 18) of the diary device (10) and/or
wherein the voice messages are, in particular automatically, transformed into text by the diary device (10) and are stored as text together with time stamps in the memory (13, 16, 18) of the diary device (10).

4. Method according to claim 3, wherein keywords and/or numeric values comprised in the voice messages are identified and converted into text by the diary device (10) and are stored as text together with a time stamp, in the memory (13, 16, 18) of the diary device (10).

5. Method according to one of the preceding claims, wherein the diary device (10) which is used comprises or is connected with means for testing the glucose level in the blood of a patient, and wherein upon a testing of the glucose level, the test result is automatically stored in the diary device (10) together with a time stamp.

6. Method according to one of the preceding claims, wherein the diary device (10) which is used comprises or is connected with means for administration of insulin (9), in particular with an insulin pen or an insulin pump, and wherein upon an in particular unscheduled administration of insulin, the dose of insulin administered is automatically stored in the diary device (10) together with a time stamp.

7. Method according to one of the claims 5 to 6, wherein the means for testing the glucose level in the blood of a patient (2) and/or the means for administration of insulin (9) are controlled via the diary device (10).

8. Method according to one of the claims 5 to 7, wherein, based at least partially on data entered into the diary device as voice messages, a specific amount of insulin to be administered is calculated and the administration thereof is initiated via the diary device (10).

9. Method according to one of the preceding claims, wherein informations about malfunctions or defects of the diary device (10) and/or of connected devices and means are stored in the diary device (10) together with time stamps, and in addition, explanatory informations relating to the malfunctions or defects are entered into the diary device (10) as voice message and are stored in the diary device as voice message and/or as text together with a separate time stamp or with the related information about a malfunction or defect.

10. Diary device (10) for keeping diary of a of a long-term testing of the glucose levels in the blood of a patient and/or of an insulin therapy of a patient suffering from diabetes,, in particular for use in the method according to one of the preceding claims, comprising:
a) a memory (13, 16, 18) for storing data data representing measured blood glucose levels and/or the administrated doses of insulin
and supplementary data directly related to the measured blood glucose levels and/or an administrated doses of insulin together with separate or common time stamps;
b) a clock circuitry (14) for generating the time stamps; and
c) a voice recording unit (6) for entering at least a part of the supplementary data, which are to be stored in the memory (13, 16, 18), into the diary device (10) by speeking of voice messages.

11. Diary device (10) according to claim 10, further comprising a memory (13, 16, 18) for storing in addition supplementary data not directly related to the measured blood glucose levels and/or an administrated doses of insulin, together with time stamps, and in particular comprising a voice recording unit (6) for entering at least a part of the supplementary data not directly related to the measured blood glucose levels and/- or an administrated doses of insulin, which are to be stored in the memory (13, 16, 18), into the diary device (10) by speeking of voice messages.

12. Diary device according to one of the claims 10 to 11, wherein the device is designed in such a manner that the voice messages can be stored in the memory (13, 16, 18) together with time stamps and/or wherein the device comprises a text recognition unit (24) for voice-to-text transformation of the entered voice messages into text to be stored in the memory (13, 16, 18) with time stamps.

13. Diary device (10) according to one of the claims 10 to 12, wherein the text recognition unit (24) is adapted to identify and transform into text only keywords and/or numeric values identified in the entered voice messages.

14. Medical device (1, 19) comprising a diary device (10) according to one of the claims 10 to 13 and further comprising means for testing the glucose level in the blood of a patient (2) and/or further comprising means for administration of insulin (9), in particular an injection module for insulin, wherein the diary device (10) is linked together with the means for testing the glucose level (2) and/or with the means for administration of insulin (9) in such a manner that, upon a testing of the glucose level and/or upon an in particular unscheduled administration of insulin, the test result and/or the dose of administered insulin can in particular automatically be stored in the memory (13, 16, 18) of the diary device (10) together with a time stamp.

15. Medical device (1, 19) according to claim 14, wherein the diary device (10) and the means for testing the glucose level in the blood of a patient (2) and/or the means for administration of insulin (9) are contained in separate housings (5) and are linked with each others by means of in particular wireless interfaces.

16. Medical device (1, 19) according to one of the claims 14 to 15, at least with an injection module for administration of insulin, further comprising means for computation of a dose of insulin to be administered (25) based at least partially on data entered into its diary device as voice messages.

17. Medical device (1, 19) according to one of the claims 14 to 15, wherein the medical device (1, 19) is designed in such a manner that the means for testing the glucose level in the blood of a patient (2) and/or the means for administration of insulin (9) can be controlled from a housing (5) containing the diary device (10).

18. Medical device (1, 19) according to one of the claims 10 to 17, wherein the diary device (10) is adapted to store informations about malfunctions or defects of the diary device (10), of the means for testing the glucose level in the blood of a patient (2) and/or of the means for administration of insulin (9) in its memory (13, 16, 18) together with time stamps, and is furthermore adapted for entering additional explanatory informations relating to the malfunctions or defects by speeking of voice messages and for storing them as voice messages and/or text in the memory (13, 16, 18) of the diary device (10), together with separate time stamps or with the related information about a malfunction or defect.

19. System comprising one or several diary devices and/or medical devices (1, 19) according to one of the claims 10 to 18 and further comprising an external device (29) to which the data stored in the memories of the devices can be downloaded via a wired or wireless interface (15), for analysing said data.
